# EUROPEAN PATENT APPLICATION

(11) **EP 1 188 749 A1**
(43) Date of publication of application: **20.03.2002**
(21) Application number: 00940778.4
(22) Date of filing: 21.06.2000
(51) Int. Cl.: C07D 221/04

(54) **PROCESS FOR THE PREPARATION OF INTERMEDIATE COMPOUNDS OF DRUGS**

(30) Priority: 22.06.1999 JP 17480299
(71) Applicant: ONO PHARMACEUTICAL CO., LTD., Osaka-shi, Osaka 541-8526 (JP)
(72) Inventor: KUWABE, Shin-itsu, Fukui 913-0032 (JP); KUSUDA, Shinya, Osaka 618-8585 (JP); HASHIMOTO, Shinsuke, Fukui 913-0032 (JP)
(74) Representative: Henkel, Feiler, Hänzel
(86) International application number: JP0004052
(87) International publication number: WO0078722

(57) **Abstract**

The present invention relates to a process for preparing selectively compound (I), (1S, 5S, 6R, 7R)-2-aza-7-chloro-5-methyl-3-oxobicyclo[4.1.0]heptane, by preparing compound (II) from compound (V) through compound (III) via a series of reactions, and by crystallizing the obtained compound (II); and the novel intermediate compound represented by the structural formula (III) and hydrate salts thereof.

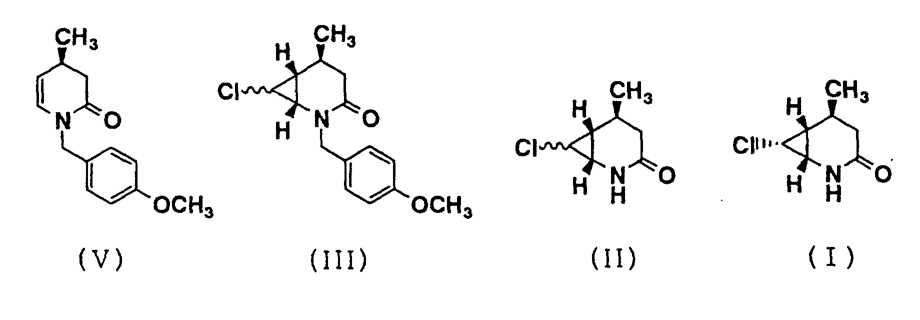

## Description

### TECHNICAL FIELD

This invention relates to the process for the preparation of intermediate compounds useful as pharmaceuticals represented by formula (X): 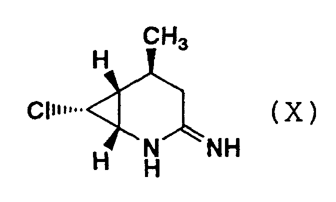 and novel intermediate compound.

More specifically, the invention relates to the process for the preparation of the intermediate compound of pharmaceuticals represented by formula (I), (1S, 5S, 6R, 7R)-2-aza-7-chloro-5-methyl-3-oxobicyclo[4.1.0]heptane, by preparing compound (II) from (45)-1-(4-methoxybenzyl)-4-methyl-5,6-dehydro-2-piperidone represented by following formula (V) via compounds represented by formula (IV), (III) and (II), then by selective crystallizing the compound (II), and the novel compound which is the intermediate of the above-mentioned process for the preparation represented by formula (III).

### BACKGROUND ART

It is disclosed in EP870763 that the compounds useful as pharmaceuticals represented by formula (A): 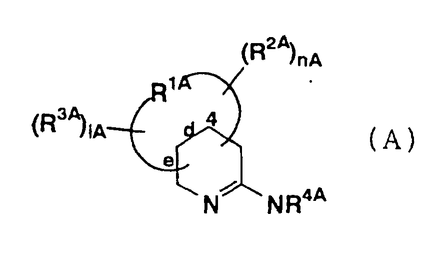 wherein -R^{1A}- represents a 3- or 4-membered carbocyclic ring together with carbon atom or atoms to which it is bonded, the carbocyclic ring being condensed side d or e of the piperidine ring or bonded to the 4-position of the piperidine ring through a spiro-union, R^{2A} represents a C1∼6 alkyl group, R^{3A} represents a C1∼6 alkyl group, a C2∼6 alkenyl group or halogen atom, R^{4A} represents a hydrogen atom, an amino-C1∼4 alkyl or a carbocyclic ring-C1∼4 alkyl group which may be substituted with an amino-C1∼4 alkyl group, iA represents 0 or an integer of 1 to 3, nA represents 0 or an integer of 1 to 3, and the plural R^{2A}s or R^{3A}s are the same or different
and the process for preparation thereof.

In the above-mentioned specification, it is disclosed that the process for the preparation of compounds represented by formula (A-1): 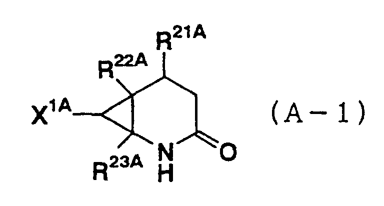 wherein R^{21A} represents a hydrogen atom or C1∼6 alkyl group, R^{22A} represents a hydrogen atom or C1∼6 alkyl group, R^{23A} represents a hydrogen atom or C1∼6 alkyl, X^{1A} represents a halogen atom
and compounds represented by formula (A-2): 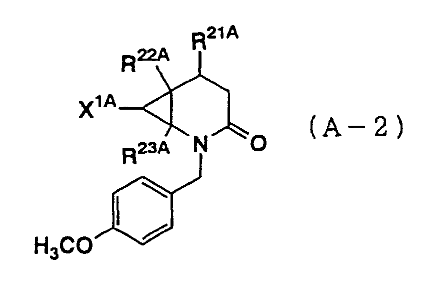 wherein all symbols are the same meaning as hereinbefore defined as intermediate of compounds represented by formula (A).

Its process for the preparation is shown in reaction schemes (A) and (B).

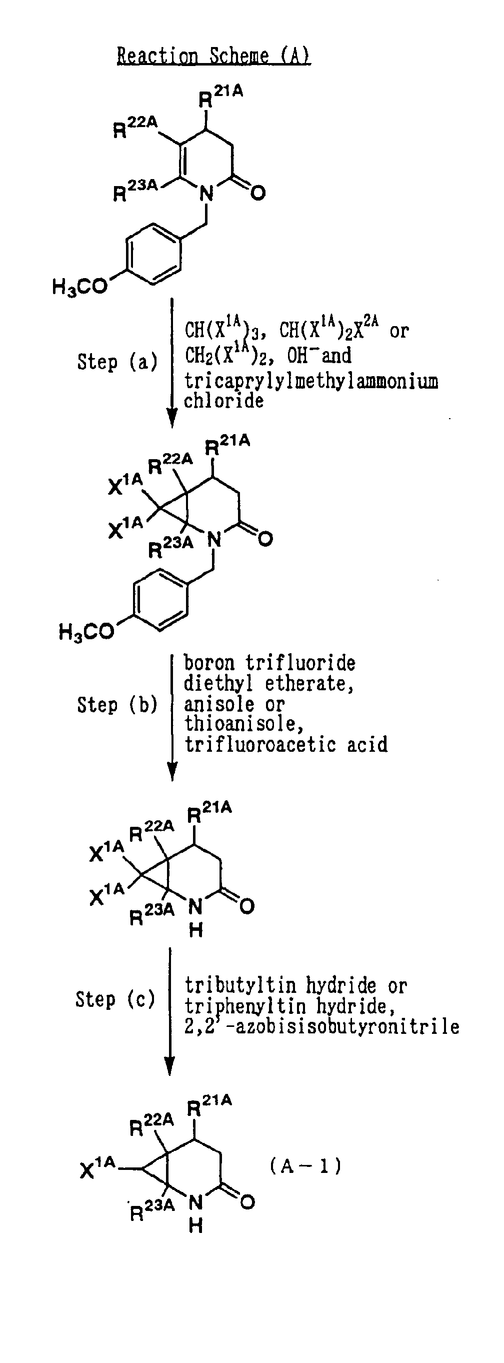

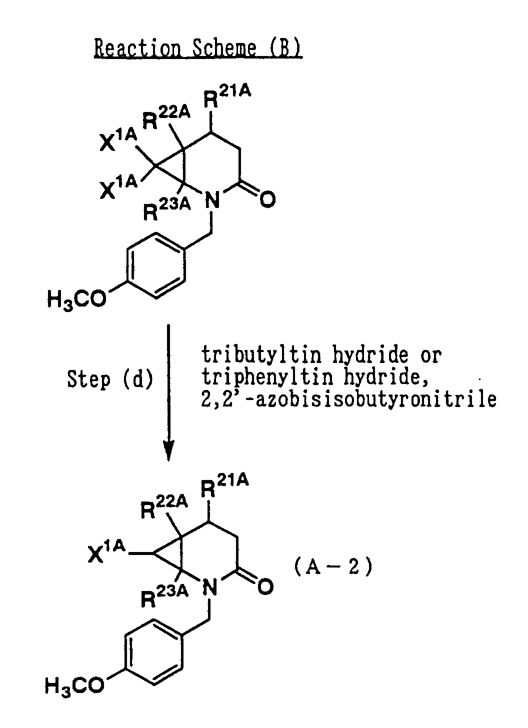

In the above-mentioned specification, it is disclosed that the process for the preparation of the compound represented by formula 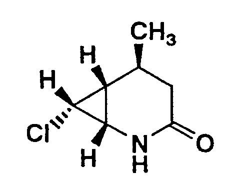 as a concrete example of the formula (A-1) in Reference example 7.

On the other hand, the concrete example of the compound represented by the formula (A-2) is not disclosed in the above-mentioned specification.

In addition, reactions described in above-mentioned reaction schemes (A), (B) and Reference example 7 in above-mentioned specification have some problems in safety, reactivity, ease in operations and cost, etc.

More specifically, (1) The step (a) is dangerous reaction because of difficulty to control reaction temperature. Moreover, tarry by-product, which is insoluble in water or most of organic solvent, is formed in the step (a). The by-product can not be removed by simple method like extraction, etc., so purification using column chromatography is necessary. Washing of the reaction vessel to which the by-product adhered is very difficult and troublesome.
(2) In the step (b), though expensive boron trifluoride diethyl etherate is used, it is fuming reagent and difficult to treat. Also, heating is necessary in this reaction, but it takes long reaction time because low boiling point of ether prevents to rise the reaction temperature. This problem becomes serious as it tries to synthesize the compound voluminously.
(3) In the step (b), anisole is used for trapping eliminated p-methoxybenzyl group. And this reaction takes long reaction time, and it has low yield and low reactivity with by-product. Moreover, the removing process of anisole trapping p-methoxybenzyl group is troublesome; for example, purification using column chromatography is necessary. Although BHT (2,6-di-t-butyl-4-methylphenol) is known as the reagent improved in reactivity, which can be replaced with anisole, it also needs purification by column chromatography.
(4) It is described that the step (b) can be carried out with thioanisole or trifluoroacetic acid. But these reactions are low yield and form many by-products.
(5) The step (c) and (d), using tributyltin hydride or triphenyltin hydride, are a chain reaction where radicals react successively, which are generated by 2,2'-azobisisobutyronitrile, and therefore control of both rate of the reaction and generation of heat are difficult, and heat might go beyond control. Moreover, it is necessary to remove poisonous tin in the reaction system completely. Separation and purification using column chromatography are necessary to obtain the compound having desired stereo configuration.

### DISCLOSURE OF THE INVENTION

A variety of investigations have been carried out to synthesis the compound promising as pharmaceuticals represented by the formula (X).

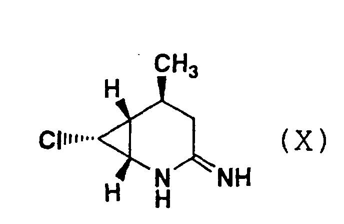

As a result, the present inventors have found the simple and easy process for the preparation of the intermediate compound represented by formula (I) and the novel intermediate compound represented by formula (III).

That is, the invention relates to the process for the preparation of (1S, 5S, 6R, 7R)-2-aza-7-chloro-5-methyl-3-oxobicyclo[4.1.0]heptane represented by formula (I): 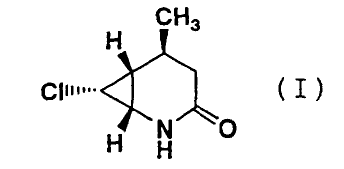 by preparing compound represented by formula (II): 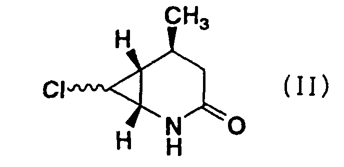 from (4S)-1-(4-Methoxybenzyl)-4-methyl-5,6-dehydro-2-piperidone represented by formula (V) through the novel intermediate compound represented by formula (III): 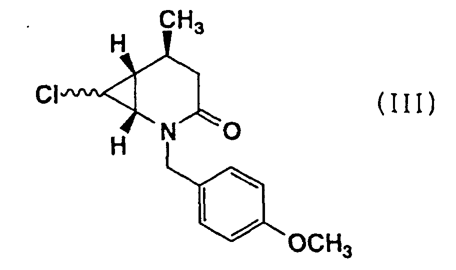 and selectively crystallizing the compound (II) and separating, and the novel intermediate compound of the process represented by formula (III).

The compound represented by formula (III): 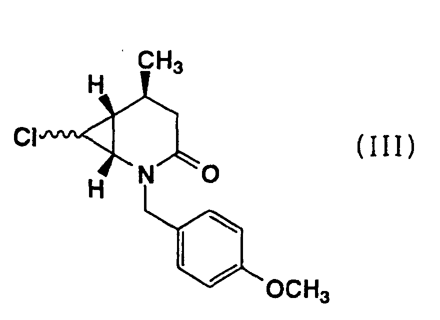 is included in the range of the formula (A-2) in the above-mentioned specification, but there is no concrete description in the above-mentioned specification. That is, the compound represented by formula (III) is the novel compound in the course of the investigations to search effective process for the preparation of the compound represented by formula (I).

The process for the preparation of the present invention is shown in reaction scheme 1.

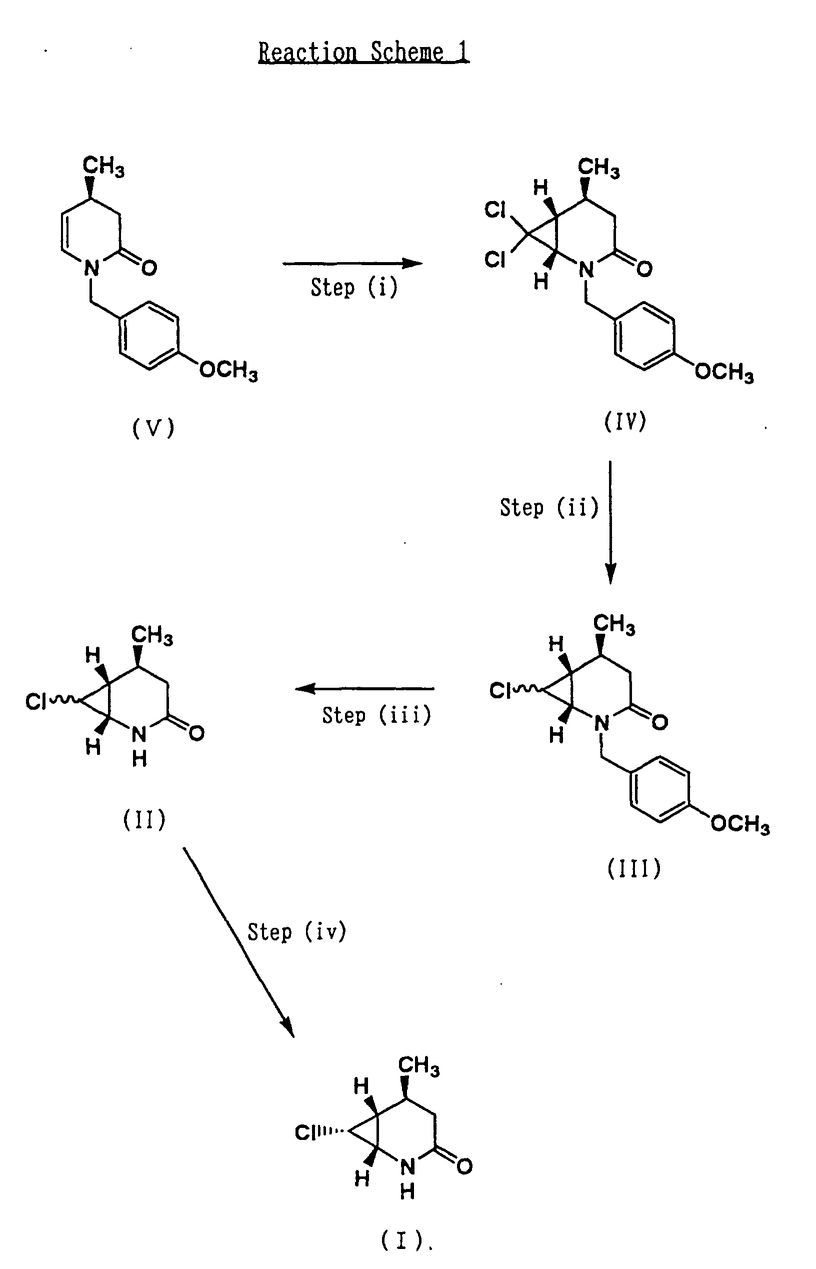

The step (I) is ring formation reaction and may be carried out, for example, in the presence of base (e.g., sodium methoxide, sodium ethoxide, sodium t-butoxide, potassium methoxide, potassium ethoxide or potassium t-butoxide), by using trichloroacetic acid derivatives (e.g., trichloroacetic acid, methyl trichloroacetate or ethyl trichloroacetate), in an organic solvent (e.g., toluene, ether series (e.g., dimethyl carbonate, diethyl carbonate, dimethoxyethane, tetrahydrofuran or diethyl ether) or a mixture thereof) at a temperature of from 0 to 40 °C.

The step (ii) is dechlorination reaction and may be carried out, for example, by using zinc (e.g., zinc dust, zinc foil or zinc granule) and amine (e.g., ethylenediamine or diethylenetriamine), in a water-soluble organic solvent (e.g., methanol, ethanol, isopropanol, butanol or ethyleneglycol) or mixture of water at a temperature of from 0 degree to reflux temperature. This reaction may be preferably carried out in the presence of a divalent lead compound (e.g., lead (II) acetate).

The step (iii) is deprotection reaction and may be carried out, for example, by using an acid (e.g., methanesulfonic acid or sulfuric acid) in an organic solvent (e.g., toluene or hexane) at a temperature of from 90 °C to reflux temperature.

The step (iv) is selective crystallization and separation by filtration. For example, it is carried out by adding hydroxide of alkali metal (e.g., sodium hydroxide, potassium hydroxide or lithium hydroxide), hydroxide of alkaline earth metal (e.g., magnesium hydroxide or calcium hydroxide) or carbonate (e.g., sodium bicarbonate, sodium carbonate or potassium carbonate) or an aqueous solution thereof or a mixture thereof to the mixture at a temperature of from -20 to 40 °C, and then, the appeared crystal is collected by suction and washed with water.

The each steps hereinbefore described may be preferably carried out in an atmosphere of inert gas.

By the processes of (i)∼(iv), the desired compound can be obtained as crystal.

The raw material compound represented by formula (V) is known, and may be prepared by the methods of the following reaction scheme 2 (tokukaihei 11-209345).

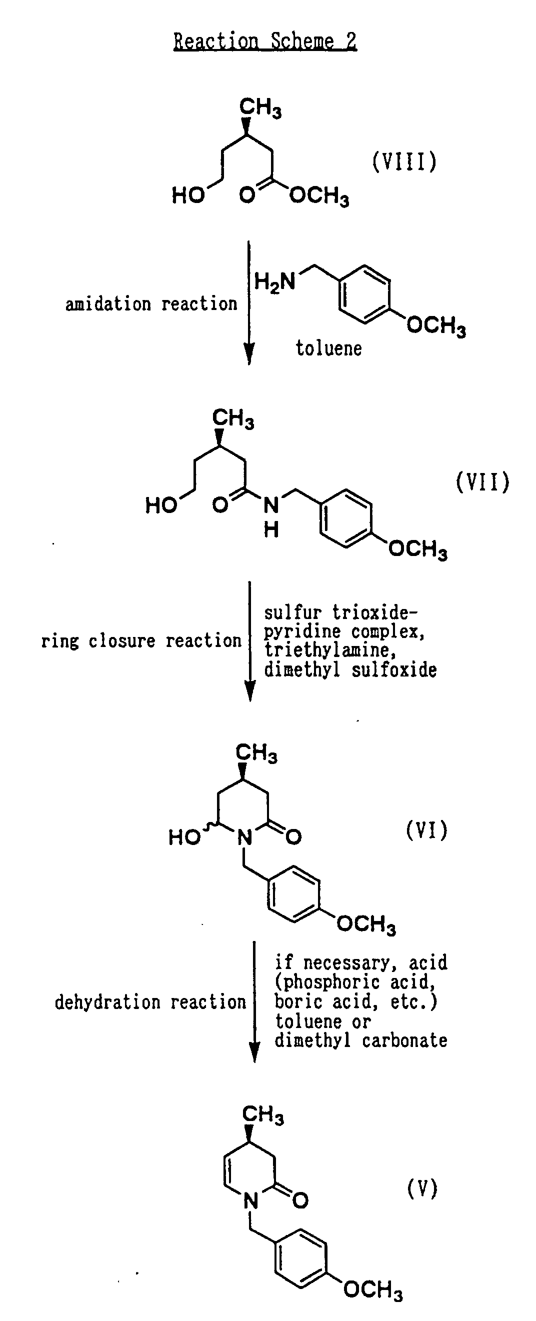

In the reaction scheme 2, the compound using as starting material is known (tokukaihei 11-209345, Example 1).

The other starting materials and each agent in the present invention are known *per se*, or may be prepared by conventional method.

According to the process for the preparation of the present invention, (1S, 5S, 6R, 7R)-2-aza-7-chloro-5-methyl-3-oxobicyclo[4.1.0]heptane represented by formula (I): 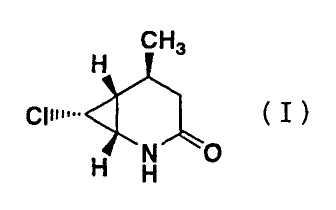 is selectively manufactured by easy operations; it is excellent in safety, reactivity, ease in operations and cost, etc.

The feature of the process for the preparation of the present invention is mentioned concretely on the basis of the reaction scheme 1 hereinbefore described.

The step (i) is the reaction, which described in EP870763, and when the operation is carried out according to the specification, the reaction isdangerous because of difficulty of thermally control. Moreover, tarry by-product is formed in the reaction, so purification using column chromatography is necessary to remove it. The reaction is also troublesome in after-treatment.

However, the present inventors have found that the use of trichloroacetic acid derivative (e.g., trichloroacetic acid, methyl trichloroacetate or ethyl trichloroacetate) which defers from the agents described in EP870763, can be accomplished easy control of temperature and safe reaction. Moreover, because tarry by-product is not generated, complicated removing operation is unnecessary, and the yield is improved from 60 % (EP870763, Example 1) to 73 %.

The step (ii) is the reaction that the compound represented by formula (III) can be obtained effectively by safe method without an over-evolution of heat using inexpensive agent. And impurity can be removed by easy operation like filtration and extraction.

As regard this step, it is known that additive (e.g., acetic acid, hydrochloric acid, sulfuric acid, potassium hydroxide or sodium hydroxide) was added to zinc in the reaction. But these additives have no effect to give chloro-form having desired stereoconfiguration (R-form) in the present invention with high ratio.

On the other hand, addition of amine (e.g., ethylenediamine or diethylenetriamine) to zinc (e.g., zinc dust, zinc foil or zinc granule) gives improvement on stereoselectivity of reduction against chlorine atom, so the chloro-form having desired stereo configuration (R-form) can be obtained with high ratio. In the reaction system, addition of divalent lead compound (e.g., lead (II) acetate) is preferable. By adding the divalent lead compound, the effect of stereoselectivity on reduction against chlorine atom is stabilized and improved. The preferable ratio of additive lead compound against the compound represented by formula (IV) is 0.2 mol%∼20 mol%.

These beneficial facts to obtain the desired compound are found for the first time by the inventors in the present invention.

The step (iii) can be carried out using inexpensive reagent in a short time, without expensive reagent, which has problem that it prevents on increase in reaction temperature. On removing operation of the by-product derived from eliminated p-methoxybenzyl group, column chromatography is unnecessary and the by-product can be removed by extraction with an organic solvent.

The step (iv) gives (7R)-chloro form compound selectively, having desired stereo configuration, as crystal by adding alkaline solution and by easy operation like crystallization and filtration, without purification by column chromatography.

This step that provides the desired (7R)-chloro form compound by crystallization from the mixture of stereoisomers without purification by column chromatography is found for the first time by the inventors in the present invention.

In each reaction described in the present specification, reaction products may be purified by conventional techniques. For example, purification may be carried out by distillation at atmospheric or reduced pressure, high performance liquid chromatography, thin layer chromatography or column chromatography using silica gel or magnesium silicate, by washing or recrystallization, etc. Purification may be carried out after each reaction, or after a series of reactions.

The compound represented by formula (III) of the present invention in the present specification may be converted into the corresponding hydrate by conventional means.

In the present invention, as may be easily understood by those skilled in the art, the symbol 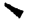 indicates that the substituent attached thereto is in front of the sheet (β -position), unless otherwise specified, the symbol 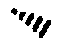 indicates that the substituent attached thereto is behind the sheet (α-position), unless otherwise specified, the symbol 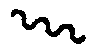 indicates that the substituent attached thereto is β-position or α-position, or the mixture of the compounds that the substituent attached thereto is β-position and αposition, the symbol 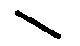 indicates the mixture of the compounds that the substituent attached thereto is β-position and α-position.

Unless othewise specified, all isomers are included in the present invention. For example, Isomers generated by the existence of asymmetric carbon atom(s) (e.g., R, S isomers, α, β isomers, enantiomers, diastereomers), optically active isomers having optically rotatory power (D, L, d, I isomers) are all included in the present invention.

### INDUSTRIAL APPLICABILITY

This invention offers the process for the preparation of the compound represented by formula (I), (1S, 5S, 6R, 7R)-2-aza-7-chloro-5-methyl-3-oxobicyclo[4.1.0]heptane, by preparing compound (II) from (4S)-1-(4-methoxybenzyl)-4-methyl-5,6-dehydro-2-piperidone represented by the formula (V) via compounds represented by formula (IV), (III) and (II), then by selective crystallizing the compound (II), and the novel compound, intermediate of the process for the preparation represented by the formula (III).

The process for the preparation in the present invention is the excellent one without the problems of prior art in safety, reactivity, ease in operations and cost, etc.

### BEST MODE FOR CARRYING OUT THE INVENTION

The following reference examples and examples illustrate the present invention, but do not limit the present invention.

The solvents in the parentheses show the developing or eluting solvents and the ratios of the solvents used are by volume in chromatographic separations or TLC.

The solvents in the parentheses in NMR show the solvents used in measurement.

### Reference example 1

### (3R)-5-Hydroxy-N-(4-methoxybenzyl)-3-methylvalerylamide

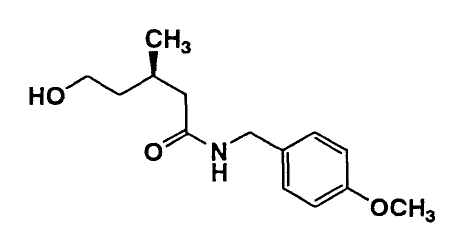

To a mixture of methyl (3R)-5-hydroxy-3-methylpentanoate (1780 g) and toluene (25 L) in reaction vessel was added 4-methoxybenzylamine (1800 ml) with stirring and the mixture was refluxed for 1.5 hours (the temperature of the reaction mixture at the beginning of reflux is ca. 110°C and its temperature after 1.5 hours is ca. 105 °C). Toluene (12.5 L) was removed from the mixture and the residue was refluxed for additional 1.5 hours. To the mixture was added 4-methoxybenzylamine (125 ml) and the mixture was refluxed for ca. 1 hour. After the reaction was completed, toluene was removed under reduced pressure. The residue was diluted with ethyl acetate (25 L) and washed with 1N hydrochloric acid (5 L, twice) and a saturated aqueous solution of sodium chloride (4 L). The aqueous solution were combined and extracted with ethyl acetate (4 L, 3 times). The organic layers were combined, dried over anhydrous magnesium sulfate and filtered. The solution was concentrated under reduced pressure to give the title compound (3.11 kg; 100% up) having the following physical data. The obtained crude crystal was used to next reaction without further purification.
TLC: Rf 0.40 (n-Hexane:Ethyl acetate=1:1);
Mass: (APCl, Pos., 40V) 252 (M+H)⁺, 136, 121;
NMR (CDCl₃): δ 7.24-7.14 (2H, m), 6.90-6.82 (2H, m), 6.03 (1H, br), 4.36 (2H, d, J = 5.6 Hz), 3.79 (3H, s), 3.72-3.59 (2H, m), 2.99 (1H, br), 2.29-2.01 (3H, m), 1.59-1.46 (2H, m), 0.97 (3H, d, J = 6.6 Hz).

### Reference example 2

### (4R)-6-Hydroxy-1-(4-methoxybenzyl)-4-methyl-2-piperidone

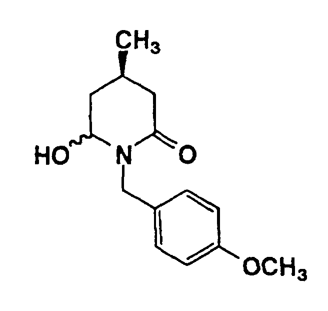

To a mixture of the compound prepared in Reference example 1 (3.07 kg) and dimethyl sulfoxide (16.3 L) was added triethylamine (8.5 L) with stirring and the mixture was cooled to below 20 °C. To the mixture was added slowly sulfur trioxide-pyridine complex (5.83 kg) keeping the temperature below 40 °C. The mixture was stirred at room temperature for 30 minutes. The reaction mixture was poured into ice-water (130 L), added ethyl acetate (30 L) and stirred for 3 minutes. After separation, aqueous layer was extracted with ethyl acetate (30 L, 3 times). The organic layer was washed with 2N hydrochloric acid (24 L, twice), water (24 L), a saturated aqueous solution of sodium bicarbonate (24 L), and a saturated aqueous solution of sodium chloride (24 L), successively, dried over anhydrous magnesium sulfate (1 kg), filtered and concentrated under reduced pressure. Crystal was collected by suction from slurry concentrate and washed with ice-cooled toluene (first crop). The mother liquid was concentrated under reduced pressure, and allowed to stand under ice-cooling to give crystalline. After crystallization, to the mixture was added ice-cooled toluene (300 ml) and the mixturewas stirred. The crystalline was collected by suction, washed with ice-cooled toluene with stirring, and then washed with ice-cooled toluene (600 ml, twice) and n-hexane (400 ml) (second crop). The first crop and second one were combined and dried under reduced pressure to give the title compound (1071.1 g; 35.2 %) having the following physical data.
TLC: Rf 0.64 (Ethyl acetate);
Mass: (APCl, Pos., 20V) : 250 (M+H)⁺;
NMR (CDCl₃): δ 7.26-7.18 (2H, m), 6.89-6.80 (2H, m), 5.02-4.90 (2H, m), 4.32 (1H, d, J = 14.6 Hz), 3.79 (3H, s), 2.71-2.54 (2H, m), 2.44-2-20 (1H, m), 2.09-1.86 (2H, m), 1.47 (1H, ddd, J = 3.6, 12.8, 13.6 Hz), 1.01 (3H, d, J = 6.6 Hz).

### Reference example 3(1)

### (4S)-1-(4-Methoxybenzyl)-4-methyl-5,6-dehydro-2-piperidone

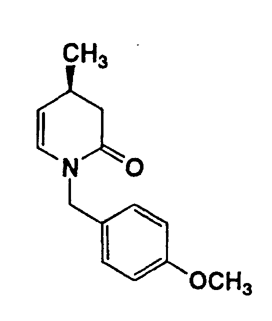

A suspension of the compound prepared in Reference example 2 (7300 g) in toluene (73 L) was refluxed for 1 hour. Toluene (ca. 37 L) was removed from the reaction mixture. Then toluene (37 L) was added to the mixture and removed toluene (ca.37 L) again. This operation was repeated once more. The content of water in the concentrate was less than 0.1%, so the reaction mixture was concentrated under reduced pressure to give the title compound. The title compound was used to next reaction without further purification.

### Reference example 3(2)

### (4S)-1-(4-Methoxybenzyl)-4-methyl-5,6-dehydro-2-piperidone

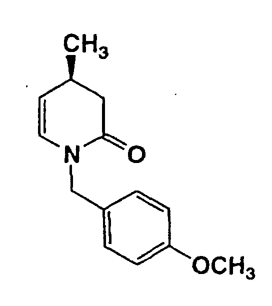

A mixture of the compound prepared in Reference example 2 (25 g), dimethyl carbonate (250 ml) and phosphoric acid (393 mg) was refluxed for ca. 1 hour. The reaction solution was concentrated under reduced pressure to give the title compound.

### Example 1

### (1S, 5S, 6R)-2-Aza-7,7-dichloro-2-(4-methoxybenzyl)-5-methyl-3-oxobicyclo[4.1.0]heptane

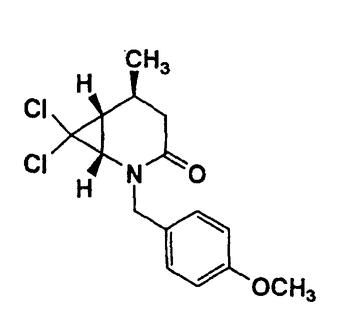

To the compound prepared in Reference example 3(1) was added dimethyl carbonate (30 L) and then the mixture was concentrated. The operation wasrepeated twice. To the residue was added dimethyl carbonate (60 L). The reaction solution was cooled with water to below ca. 25 °C and then added sodium ethoxide (9962 g). The reaction solution was cooled with water, added dropwise ethyl trichloroacetate (16820 g; corresponding to ca 12.1 L) with keeping the temperature 20∼25 °C under cooling with water and stirred for ca. 1 hour. The reaction solution was added water (59 L) and extracted with t-butyl methyl ether (59 L). The aqueous layer was extracted with t-butyl methyl ether (29 L). The organic layers were combined and washed with 1N aqueous solution of sodium hydroxide (29 L), water (29 L) and a saturated aqueous solution of sodium chloride (59 L), successively, and then concentrated under reduced pressure. The residue was purified by column chromatography on silica gel toluene:ethyl acetate=10:1). The obtained fraction was concentrated under reduced pressure and the residue was azeotroped with methanol (15 L) twice to give the title compound (6738 g; 73 %) having the following physical data.
TLC: Rf 0.43 (n-Hexane:Ethyl acetate=2:1);
Mass: (APCI, Pos., 40V) : 314 (M+H, 35Cl)⁺, 121;
NMR (CDCl₃): δ 7.33-7.22 (2H,m), 6.93 - 6.85 (2H, m), 5.44 (1H, d, J = 14.2 Hz), 3.81 (1H, d, J = 14.2 Hz), 3,81 (3H, s), 2.95 (1H,d,J = 9.8 Hz), 2.39 - 2.02 (3H, m), 1.76 (1H, dd,J = 5.4, 10.0 Hz), 1.25 (3H, d,J = 6.2 Hz).

### Example 2(1)

### (1S, 5S, 6R)-2-Aza-7-chloro-2-(4-methoxybenzyl)-5-methyl-3-oxobicyclo[4.1.0]heptane

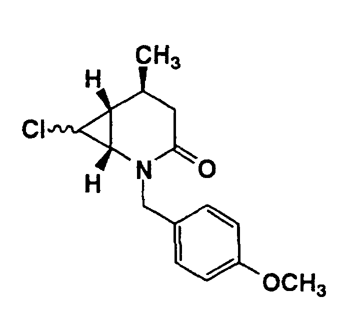

To a solution of the compound prepared in Example 1 (2936 g) in methanol (11.8 L) was added purified water (2.95 L). To the solution was added zinc dust (94.6%, Wako)(1223 g) and ethylenediamine (5620 g) and the mixture was stirred at reflux temperature for 3 hours. The end point of the reaction was confirmed by HPLC analysis (ratio of the product was (7R)-chloro form:(7S)-chloro form=78.9%:15.7%≒ 5:1). The reaction solution was cooled to room temperature, added methanol (5.9 L) and stirred for 5 minutes. Moreover, this operation hereinbefore described was repeated once more using the compound prepared in Example 1 (2936 g), then the obtained solution was combined with previous one. The combined solution was filtered. The impurity was filtered off and washed with methanol (11.7 L). The filtrate was concentrated under reduced pressure. To the concentrate was added t-butyl methyl ether (35 L) and water (35 L) and then extracted. The aqueous layer was extracted with t-butyl methyl ether (35 L). The organic layers were combined, washed with 1M hydrochloric acid (5.9 L, twice), water (11.7 L) and a saturated aqueous solution of sodium chloride (11.7 L), successively, and concentrated under reduced pressure. The residue was azeotroped with toluene (11.7 L) twice to give the title compound (4338 g; 83 %) having the following
physical data.
TLC: Rf 0.40 (n-Hexane: Ethyl acetate=2:1);
NMR (CDCl₃): δ 7.30-7.20 (m, 2H), 6.90-6.80 (m, 2H), 5.50 (d, J = 14 Hz) and 4.75 (d, J = 14 Hz, 1H), 4.42 (d, J = 14 Hz) and 3.62 (d, J = 14 Hz, 1H), 3.80 (s, 3H), 3.25 (dd, J = 5.3, 3.5 Hz) and 2.65 (dd, J = 2.0, 3.6 Hz, 1H), 2.65 (dd, J = 6.8, 3,5 Hz) and 2.72 (dd, J = 7.2, 2.0 Hz, 1H), 2.40-2.10 (m, 3H), 1.45-1.10 (m, 1H), 1.20 (d, J = 4.5 Hz, 3H).

### Example 2(2)

### (1 S, 5S, 6R)-2-Aza-7-chloro-2-(4-methoxybenzyl)-5-methyl-3-oxobicyclo[4.1.0]heptane

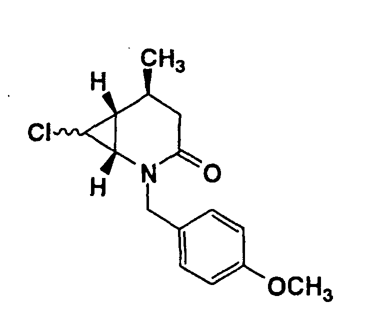

To a solution of the compound prepared in Example 1 (36.9 g) in methanol (148 ml) was added purified water (37 ml). To the solution were added zinc dust (min. 95%, Merck)(15.4 g), lead (II) acetate trihydrate (891 mg) and ethylenediamine (70.6 g) and the mixture was stirred at reflux temperature for 3 hours. The end point of the reaction was confirmed by HPLC analysis (ratio of the product was (7R)-chloro form:(7S)-chloro form=72.1%:18.8%≒ 3.8:1). The reaction solution was cooled to room temperature, added methanol (74 ml) and stirred for 5 minutes. The solution was filtered. The impurity was filtered off and washed with methanol (74 ml). The filtrate was concentrated under reduced pressure. To the concentrate was added t-butyl methyl ether (220 ml) and water (220 ml) and then extracted. The aqueous layer was extracted with t-butyl methyl ether (220 ml). The organic layers were combined, washed with 1M hydrochloric acid (37 ml, twice), water (74 ml) and a saturated aqueous solution of sodium chloride (74 ml), successively, and concentrated under reduced pressure. The residue was azeotroped with toluene (74 ml) twice to give the title compound (32.7 g; 99.6 %) having the following physical data.
TLC: Rf 0.40 (n-Hexane:Ethyl acetate=2:1);
NMR (CDCl₃): δ 7.30-7.20 (m, 2H), 6.90-6.80 (m, 2H), 5.50 (d, J = 14 Hz) and 4.75 (d, J = 14Hz, 1H), 4.42 (d, J = 14 Hz) and 3.62 (d, J = 14 Hz, 1H), 3.80 (s, 3H), 3.25 (dd, J = 5.3, 3.5 Hz) and 2.65 (dd, J = 2.0, 3.6 Hz, 1H), 2.65 (dd, J = 6.8, 3.5 Hz) and 2.72 (dd, J = 7.2, 2.0 Hz, 1H), 2.40-2.10 (m, 3H), 1.45-1.10 (m, 1H), 1.20 (d, J = 4.5 Hz, 3H).

### Example 3

### (1S, 5S, 6R, 7R)-2-Aza-7-chloro-5-methyl-3-oxobicyclo[4.1.0]heptane

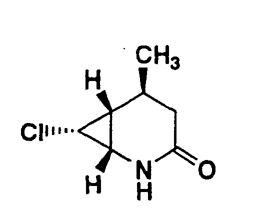

The compound prepared in Example 2(1) (4930 g) was dissolved in the mixture of methanesulfonic acid (16913 g) and toluene (14.9 L) and the mixture was refluxed for 2 hours. The reaction mixture was cooled to room temperature, diluted with cooled water (ca. 5 °C; 19.8 L), added n-hexane (29.8 L) and removed impurity. The aqueous layer was washed with n-hexane (29.8 L) and toluene (14.9 L). To the aqueous layer was added dropwise 4 mol/L aqueous solution of sodium hydroxide (44.3 L) under ice-cooling, then crystalline was appeared. The temperature of aqueous solution was cooled below 25 °C, the crystal was collected by suction and washed with water (19.8 L). The obtained crystal was dried under reduced pressure overnight at 30 °C to give the title compound (1547 g; 55 %) having the following physical data
TLC: Rf 0.39 (Ethyl acetate);
MASS (APCI, Pos., 40V): 160 (M+H, 35Cl)⁺, 124, 122;
NMR (CDCl₃): δ 6.17 (1 H, br), 3.27 (1 H, dd, J = 5.2, 7.8 Hz), 2.87 (1 H, ddd, J = 1.2, 5.2, 9.2 Hz), 2.30-2.04 (3H, m), 1.37-1.22 (1H, m), 1.24 (3H, d, J = 6.0 Hz).

### Example 4

### (1S, 5S, 6R, 7R)-2-Aza-7-chloro-3-methoxy-5-methylbicyclo[4.1.0]hept-2-ene

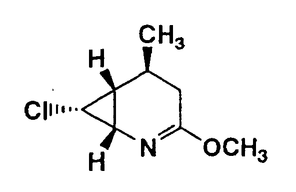

The compound prepared in Example 3 (1400 g) was dissolved in dimethyl carbonate (35 L) under an atmosphere of argon. To a solution was added Meerwein reagent (trimethyloxonium tetrafluoroborate) (2594 g) and the mixture was stirred at room temperature for 3 hours under an atmosphere of argon. To a reaction solution were added t-butyl methyl ether (35 L) and an aqueous solution of sodium bicarbonate (35 L) and the mixture was stirred. The two layers were separated and the aqueous layer was extracted with ethyl acetate. The combined organic layer was washed with an aqueous solution of sodium bicarbonate (17.5 L) and a saturated aqueous solution of sodium chloride (17.5 L), successively, dried over anhydrous magnesium sulfate (1700 g) and concentrated under reduced pressure. To the residue was added ethanol (4.4 L) and the mixture was concentrated under reduced pressure to give the title compound. The compound was used to next reaction without further purification.

### Example 5

### (1S, 5S, 6R, 7R)-2-Aza-7-chloro-3-imino-5-methylbicyclo[4.1.0]heptane monohydrochloride

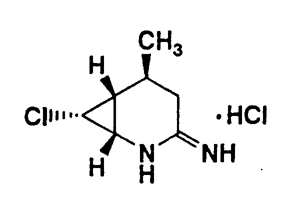

To the compound prepared in Example 4 were added ethanol (8.8 L) and ammonium acetate (2029 g) and the mixture was refluxed for 1.5 hours. The reaction solution was cooled to 5 °C, added ethyl acetate (35 L) and stirred for 10 minutes. Precipitate was filtered off and washed with ethyl acetate (8.8 L). The filtrate was concentrated under reduced pressure and the residue was dissolved in ethanol (8.8 L). The solution was cooled to 5 °C, added 4N hydrogen chloride in ethyl acetate (8.8 L) and ethyl acetate, successively, and stirred at 5 °C for 30 minutes. Appeared crystal was collected by suction, washed with ethyl acetate (8.8 L) twice and dried under reduced pressure at 25 °C overnight to give the title crude compound (1197 g; 70 %). To the crude product (983 g) were added ethanol (13 L) and ethyl acetate (2 L), the mixture was heated to the internal temperature 60-70 °C to dissolve. The solution was filtered and washed with ethanol (1 L). The filtrate was keptstanding to cool and then stirred for 30 minutes under cooling with ice. The solution was filtered and the obtained crystal was washed with ethyl acetate / ethanol (2 L / 1 L). The crystal was dried under reduced pressure to give the title compound (737 g; 75 %) having the following physical data.
TLC: Rf 0.33 (Chloroform : Methanol : Acetic acid = 10 : 1: 1);
NMR (DMSO-d₆): δ 9.80-9.65 (1H, br), 9.30-9.10 (1H, br), 8.80-8.60 (1H, br), 3.64 (1H, dd, J = 7.7, 5.5 Hz), 3.04 (1H, dd, J = 8.9, 5.5 Hz), 2.41 (2H, d, J = 8.2 Hz), 1.90-1.70 (1H, m), 1.55-1.40 (1H, m), 1.21 (3H, d, J = 6.8 Hz);
specific rotation: [α]_{D} +81.4 degrees (c = 0.160, Methanol).

## Claims

1. A process for the preparation of (1S, 5S, 6R, 7R)-2-aza-7-chloro-5-methyl-3-oxobicyclo[4.1.0]heptane represented by formula (I): 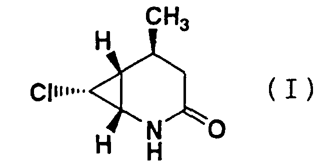 comprising subjecting (1S, 5S, 6R)-2-aza-7-chlora-5-methyl-3-oxobicyclo[4.1.0]heptane represented by formula (II): 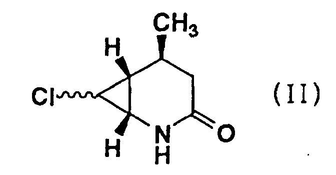 to selective crystallization and separation.

2. A process for the preparation of (1S, 5S, 6R, 7R)-2-aza-7-chloro-5-methyl-3-oxobicyclo[4.1.0]heptane represented by formula (I) according to claim 1, comprising subjecting (1S, 5S, 6R)-2-aza-7-chloro-2-(4-methoxybenzyl)-5-methyl-3-oxobicyclo[4.1.0]heptane represented by formula (III): 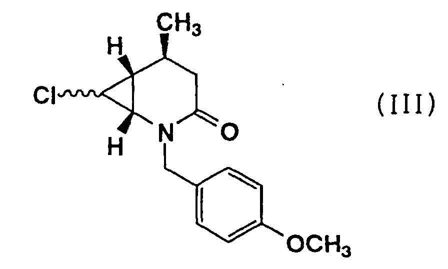 to deprotection reaction, and subjecting the resulting (1S, 5S, 6R)-2-aza-7-chloro-5-methyl-3-oxobicyclo[4.1.0]heptane represented by formula (II): 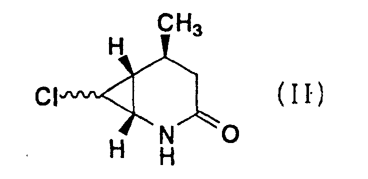 to crystallization.

3. A process for the preparation of (1S, 5S, 6R, 7R)-2-aza-7-chloro-5-methyl-3-oxobicyclo[4.1.0]heptane represented by formula (I) according to claim 1 or 2, comprising subjecting (1S, 5S, 6R)-2-aza-7,7-dichloro-2-(4-methoxybenzyl)-5-methyl-3-oxobicyclo[4.1.0]heptane represented by formula (IV): 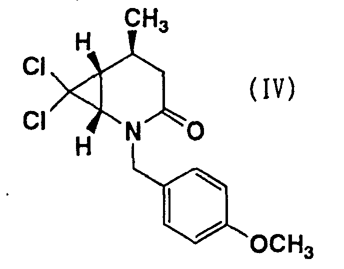 to dechlorination, subjecting the resulting (1S, 5S, 6R)-2-aza-7-chloro-2-(4-methoxybenzyl)-5-methyl-3-oxobicyclo[4.1.0]heptane represented by formula (III): 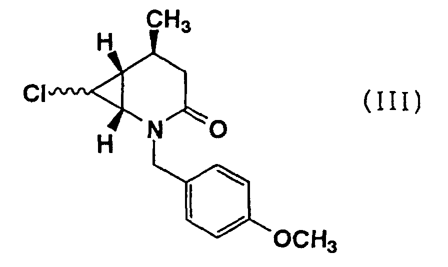 to deprotection reaction, and subjecting the resulting (1S, 5S, 6R)-2-aza-7-chloro-5-methyl-3-oxobicyclo[4.1.0]heptane represented by formula (II): 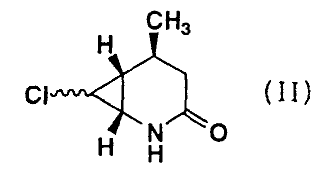 to crystallization.

4. A process for the preparation of (1S, 5S, 6R, 7R)-2-aza-7-chloro-5-methyl-3-oxobicyclo[4.1.0]heptane represented by formula (I) according to claim 1∼3, comprising subjecting (4S)-1-(4-methoxybenzyl)-4-methyl-5,6-dehydro-2-piperidone represented by formula (V): 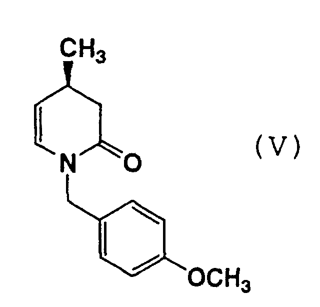 to the reaction with trichloroacetic acid derivatives in the presence of base, subjecting the resulting (1S, 5S, 6R)-2-aza-7,7-dichloro-2-(4-methoxybenzyl)-5-methyl-3-oxobicyclo[4.1,0]heptane represented by formula (IV): 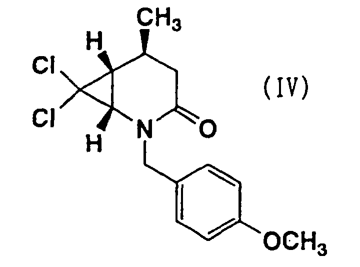 to dechlorination, subjecting the resulting (1S, 5S, 6R)-2-aza-7-chloro-2-(4-methoxybenzyl)-5-methyl-3-oxobicyclo[4.1.0]heptane represented by formula (III): 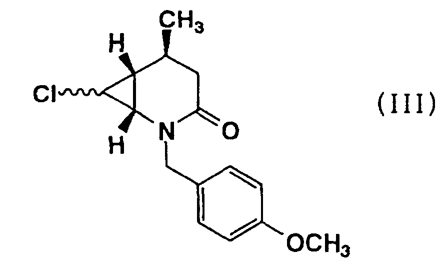 to deprotection reaction, and subjecting the resulting (1S, 5S, 6R)-2-aza-7-chloro-5-methyl-3-oxobicyclo[4.1.0]heptane represented by formula (II): 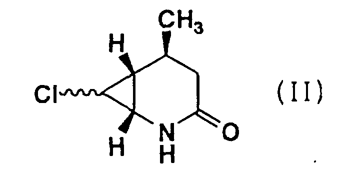 to crystallization.

5. A process for the preparation of (1S, 5S, 6R)-2-aza-7-chloro-5-methyl-3-oxobicyclo[4.1.0]heptane represented by formula (II): 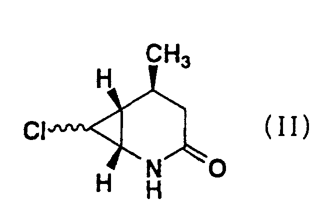 comprising subjecting (1S, 5S, 6R)-2-aza-7-chloro-2-(4-methoxybenzyl)-5-methyl-3-oxobicyclo[4.1.0]heptane represented by formula (III): 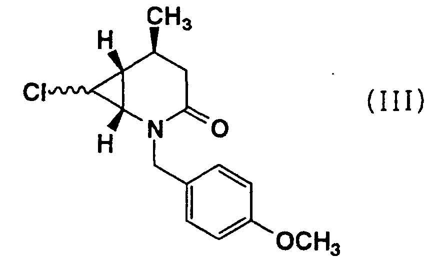 to deprotection reaction.

6. A process for the preparation of (1S, 5S, 6R)-2-aza-7-chloro-5-methyl-3-oxobicyclo[4.1.0]heptane represented by formula (II) according to claim 5, wherein methanesulfonic acid is used in deprotection reaction.

7. A process for the preparation of (1S, 5S, 6R)-2-aza-7-chloro-2-(4-methoxybenzyl)-5-methyl-3-oxobicyclo[4.1.0]heptane represented by formula (III): 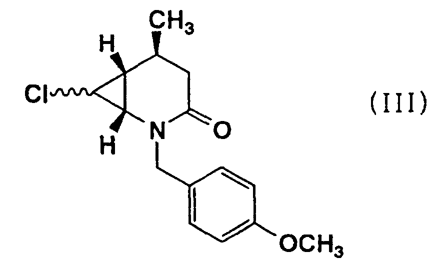 comprising subjecting (1S, 5S, 6R)-2-aza-7,7-dichloro-2-(4-methoxybenzyl)-5-methyl-3-oxobicyclo[4.1.0]heptane represented by formula (IV): 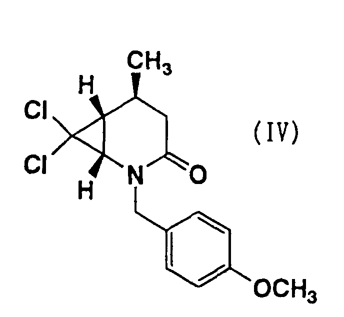 to dechlorination.

8. A process for the preparation of (1S, 5S, 6R)-2-aza-7-chloro-2-(4-methoxybenzyl)-5-methyl-3-oxobicyclo[4.1.0]heptane represented by formula (III) according to claim 7, wherein zinc, amine and, if necessary, divalent lead compound are used in dechlorination.

9. A process for the preparation of (1S, 5S, 6R)-2-aza-7,7-dichloro-2-(4-methoxybenzyl)-5-methyl-3-oxobicyclo[4.1.0]heptane represented by formula (IV): 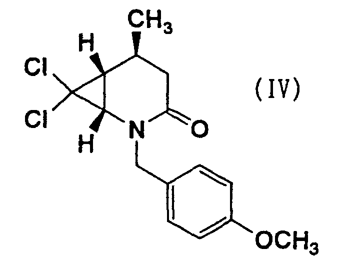 comprising subjecting (4S)-1-(4-methoxybenzyl)-4-methyl-5,6-dehydro-2-piperidone represented by formula (V): 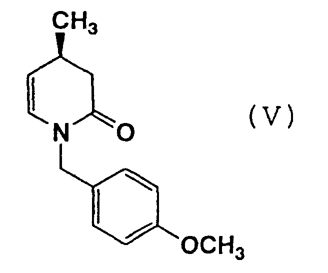 to the reaction with trichloroacetic acid derivatives in the presence of base.

10. A compound represented by formula (III): 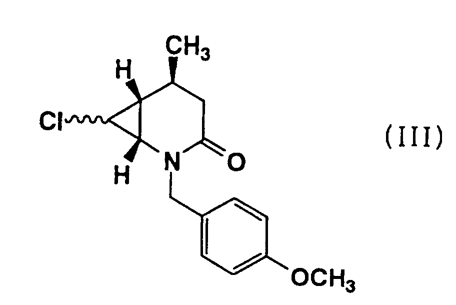 or a hydrate salt thereof.
